(19) Europäisches Patentamt
European Patent Office
Office européen des brevets

(11) Publication number: **0 210 351 B1**

(12) **EUROPEAN PATENT SPECIFICATION**

(45) Date of publication of patent specification: **21.07.93**

(51) Int. Cl.$^5$: **A61K 31/40**, A61K 49/02

(21) Application number: **86105954.1**

(22) Date of filing: **30.04.86**

(54) **Use of porphyrin derivatives in the detection and treatment of tumours.**

(30) Priority: **30.04.85 US 728786**

(43) Date of publication of application:
**04.02.87 Bulletin 87/06**

(45) Publication of the grant of the patent:
**21.07.93 Bulletin 93/29**

(84) Designated Contracting States:
**AT BE CH DE FR GB IT LI LU NL SE**

(56) References cited:
**EP-A- 0 168 832**
**WO-A-84/01382**
**DE-A- 2 809 093**

**IGUKU TO SEIBUTSUGAKU, vol. 90, no.4, April 10, 1975, pages 161-164, JP. T. YAMAMOTO: "Tumor suppression by photdynamic action with phytochloring sodium"**

**PHOTOCHEMISTRY AND PHOTOBIOLOGY, vol. 40, no. 3, 1984, pages 403-40, Pergamon Press, GB**

**D. KESSEL et al.: "Photodynamic effects: Porphyrin VS chlorin"**

(73) Proprietor: **Nippon Petrochemicals Co., Ltd.**
**Saiwai Building, 1-3-1 Uchisaiwai-cho**
**Chiyoda-ku**
**Tokyo(JP)**

(72) Inventor: **Bommer, Jerry C.**
**Rural Route No. 2 Box 516**
**Ogden Utah(US)**
Inventor: **Burnham, Bruce F.**
**127 West 900 North**
**Logan Utah(US)**

(74) Representative: **Lehn, Werner, Dipl.-Ing. et al**
**Hoffmann, Eitle & Partner Patentanwälte Arabellastrasse 4**
**W-8000 München 81 (DE)**

**EP 0 210 351 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to new compositions which are useful in photodiagnosis and phototherapy, especially in the detection and treatment of tumors and cancerous tissues in the human or animal body.

It is known to irradiate tumors and cancerous tissues in the human body with intensive light following administration of a hematoprophyrin derivative in the wavelength range of 626 to 636 nanometers to reduce and, at times, destroy the cancerous cells (see PCT published specification WO83/00811). It is also known that porphyrins, especially the sodium salt of protoporphyrins, can maintain or promote the normal functions of cells and are useful for preventing the genesis, growth, metastasis, and relapse of malignant tumors. Japanese Published Patent Application No. 125737/76 describes the use of porphyrins as tumor inhibiting agents, exemplifying etioporphyrin, mesoporphyrin, protoporphyrin, deuteroporphyrin, hematoporphyrin, coproporphyrin, and uroporphyrin.

That some of the tetrapyrroles cause intense photosensitivity in animals is well-known and has been documented in numerous articles in literature, e.g., J. Intr. Sci. Vitaminol, 27, 521-527 (1981); Agric. Biol. Chem., 46(9), 2183-2193 (1982); Chem. Abst. 98, 276 (1983) and 88, 69764m (1928).

The diagnostic and therapeutic agents of the present invention are tetrapyrrole carboxylic acids which are known per se or preparable by various procedures from naturally-occurring tetrapyrroles. The common characteristic of the present therapeutic agents is the presence of three carboxylic acid groups in the molecule.

The tetrapyrroles employed in the present invention are all known or derived by various means and various alteration procedures from natural tetrapyrroles. The naturally occurring tetrapyrroles have as their common ancestor uroporphyrinogen III, a hexahydroporphyrin reduced at the bridge positions.

The invention is directed to a diagnostic composition for detection of mammalian tumors which comprises at least one fluorescent compound of the formula:

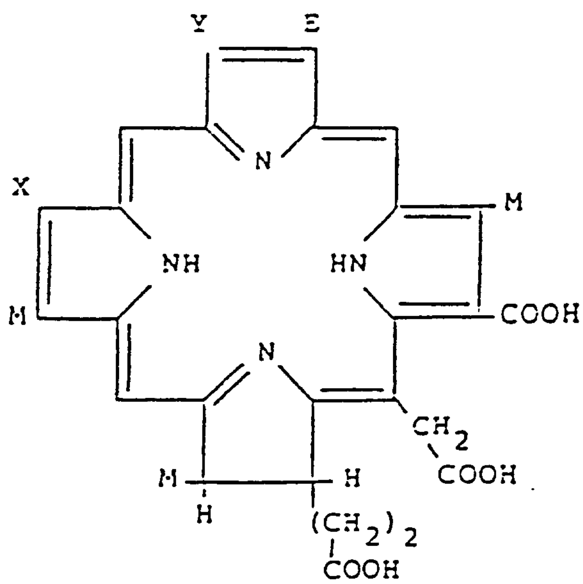

wherein:

X = H, vinyl, ethyl, acetyl or formyl;
Y = methyl or formyl;
M = methyl;
E = ethyl;

and pharmaceutically-acceptable salts thereof.

The invention also comprises a therapeutic composition for the treatment of mammalian tumors which comprises an anti-tumor effective amount of at least one fluorescent compound of the formula:

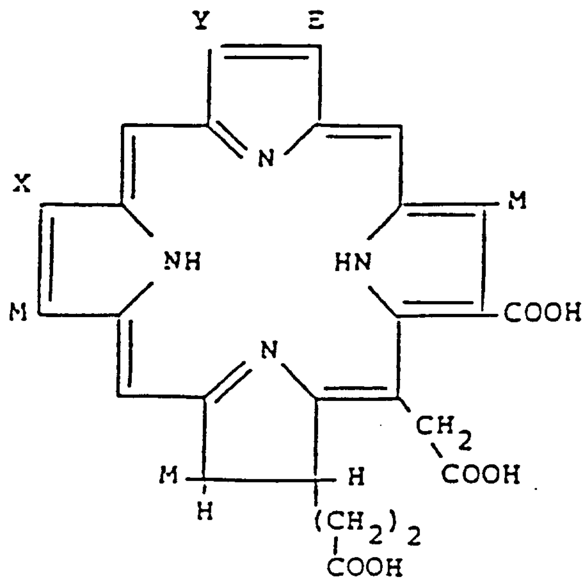

wherein:

X = H, vinyl, ethyl, acetyl, formyl;
Y = methyl or formyl;
M = methyl;
E = ethyl;

and pharmaceutically-acceptable salts thereof, with the proviso that said compound is not chlorin $e_6$.

Exemplary compounds of the tetrapyrrole classes are illustrated in Table I in which the numbered positions of the tetrapyrrole ring structure are used to designate the position of the indicated substituent. The absence of double bonds in the ring system is designated under "dihydro" with each set of numbers (ring position) indicating the absence of a double bond between the designated positions.

TABLE I - Ring Position

| PORPHYRIN | A: 1 | A: 2 | B: 6 | B: 7 | C: 11 | C: 12 | 14 | D: 16 | D: 17 | Dihydro |
|---|---|---|---|---|---|---|---|---|---|---|
| Chlorin $e_6$ | Me | V | Me | Et | Me | $CO_2H$ | Ac | H<br>Pr | H<br>Me | 16,17 |
| Mesochlorin $e_6$ | Me | Et | Me | Et | Me | $CO_2H$ | Ac | H<br>Pr | H<br>Me | 16,17 |
| Bacteriochlorin $e_6$ | Me | ACL | H<br>Me | H<br>Et | Me | $CO_2H$ | Ac | H<br>Pr | H<br>Me | 6,7<br>16,17 |
| 2-Desvinylchlorin $e_6$ (or Deuterochlorin $e_6$) | Me | H | Me | Et | Me | $CO_2H$ | Ac | H<br>Pr | H<br>Me | 16,17 |
| 2-Acetylchlorin $e_6$ | Me | ACL | Me | Et | Me | $CO_2H$ | Ac | H<br>Pr | H<br>Me | 16,17 |
| 2-Formylchlorin $e_6$ | Me | CHO | Me | Et | Me | $CO_2H$ | Ac | H<br>Pr | H<br>Me | 16,17 |
| Rhodin $g_7$ | Me | V | CHO | Et | Me | $CO_2H$ | Ac | H<br>Pr | H<br>Me | 16,17 |

Notes:

Me: $-CH_3$ (Methyl group)
Pr: $-CH_2CH_2COOH$ (Propionic acid group)
V: $-CH=CH_2$ (Vinyl group)
Et: $-CH_2CH_3$ (Ethyl group)
Ac: $-CH_2COOH$ (Acetic acid group)
ACL: $CH_3-CO-$ (Acetyl group)

The aforesaid compounds form salts with either acids or bases. The acid salts are particularly useful for purification and/or separation of the final products as are the salts formed with bases. The base salts, however, are particularly preferred for diagnostic and therapeutic use as hereindescribed.

The acid salts are formed with a variety of acids such as the mineral acids, hydrochloric, hydrobromic, nitric and sulfuric acids, and organic acids such as toluenesulfonic and benezenesulfonic acids.

The base salts include, for example, sodium, potassium, calcium, magnesium, ammonium, triethylammonium, trimethylammonium. morpholine and piperidine salts and similar such salts.

The acid and base salts are formed by the simple expediency of dissolving the selected tetrapyrrole in an aqueous solution of the acid or base and evaporation of the solution to dryness. The use of a water-miscible solvent for the tetrapyrrole can assist in dissolving the amide.

The tetrapyrroles can also be converted to metal complexes for example by reaction with metal salts, e.g., the magnesium complexes which are useful for the same purpose as the tetrapyrrole.

Photodiagnosis and Phototherapy

The compositions of the present invention are useful for the photodiagnosis and phototherapy of tumor, cancer and malignant tissue (hereinafter referred to as "tumor").

When a man or animal i.e. a mammal, having a tumor is treated with doses of a compound of the present invention and when appropriate light rays or electromagnetic waves are applied, the compound emits light, i.e. fluorescence. Thereby the existence, position and size of tumor can be detected, i.e., photodiagnosis.

When the tumor is irradiated with light of proper wavelength and intensity, the compound is activated to exert a cell killing effect against the tumor. This is called "phototherapy".

Compounds intended for photodiagnosis and phototherapy ideally should have the following properties:

(a) non-toxic at normal therapeutic dosage unless and until activated by light;

(b) should be selectively photoactive,

(c) when light rays or electromagnetic waves are applied, they should emit characteristic and detectable flourescence;

(d) when irradiated with light rays or electromagnetic waves are applied, they are activated to an extend to exert a cell killing effect against tumor; and

(e) easily metabolized or excreted after treatment.

In accordance with testing up to the present, the compounds of the present new therapeutic composition have the foregoing properties and are also characterized by reasonable solubility in water at physiological pH.

The aforesaid compounds possess greater fluorescence in tumors than do other tetrapyrroles reported in the prior art. Their use provides the best contrast in tumors compared to normal tissue around the tumor. The instant compounds absorb activating energy for phototherapy in the convenient range of 600 to 800 nanometers, with the preferred compound absorbing in the 620-760 nanometer range, i.e., light of longer wavelengths which more readily permits penetration of energy into the tumor for phototherapeutic purpose.

In present experience, the present compounds distribute more uniformly throughout the tumor permitting the use of considerably lower dosage which lessens, if not eliminates, photosensitization in the host. They also possess a more consistent fluorescence whereas some of the prior art tetrapyrroles show inconsistent fluorescence or the fluorescence varies from day to day in the host.

The instant composition can be used for diagnosis and therapeutic treatment of a broad range of tumors. Examples of tumors are gastric cancer, enteric cancer, lung cancer, breast cancer, uterine cancer, esophageal cancer, ovarian cancer, pancreatic cancer, pharyngeal cancer, sarcomas, hepatic cancer, cancer of the urinary bladder, cancer of the upper jaw, cancer of the bile duct, cancer of the tongue, cerebral tumor, skin cancer, malignant goiter, prostatic cancer, cancer of the parotid gland, Hodgkins's disease, multiple myeloma, renal cancer, leukemia, and malignant lymphocytoma. For diagnosis, the sole requirement is that the tumor be capable of selectivity fluorescing when exposed to proper light. For treatment, the tumor must be penetrable by the activation energy. For diagnosis, light of shorter wavelength is used whereas for therapeutic purposes light of longer wavelength is used to permit ready penetration of the tumor tissue. Thus, for diagnosis, light of from 360 - 760 nanometers can be used, and for treatment, from 620 - 760, depending on the individual characteristics of the tetrapyrrole.

It is necessary that the light rays be so intense as to cause the compounds to emit fluorescence for diagnosis and to exert a cell killing effect for therapy.

The source of irradiation for photodiagnosis and phototherapy is not restricted, however, but the laser beam is preferable because intensive light rays in a desired wavelength range can be selectively applied. For example, in photodiagnosis, the compound of the invention is administered to a human or animal body, and after a certain period of time, light rays are applied to the part to be examined. When an endoscope can be used for the affected part, such as lungs, gullet, stomach, womb, urinary bladder or rectum, it is irradiated using the endoscope, and the tumor portion selectively emits fluorescence. This portion is observed visually, or observed through an adapted fiber scope by eye or on a CRT screen.

In phototherapy, after administration of the dosage, the irradiation is carried out by laser beams from the tip of quartz fibers. Besides the irradiation of the surface of tumor, the internal part of the tumor can be irradiated by inserting the tip of quartz fibers into the tumor. The irradiation can be visually observed or imaged on a CRT screen.

For photodiagnosis, light of wavelengths between 360 and 760 nm. is suitable for activating the present tetrapyrrole compounds. Of course, each compound has a specific optimal wavelength of activation. A long wavelength ultraviolet lamp is particularly suitable for photodiagnosis. Similar methods for viewing of the treated tumor can be used as already described for phototherapy.

The dosages of the compounds having the present, new composition will vary depending on the desired effect, whether for diagnosis or for treatment. For diagnosis, doses of as little as 1 mg/kg will be effective, and up to about 20 mg/kg can be used. For treatment, the dose will usually approximate about 0.5 mg/kg. Of course, the dosage for either diagnosis or treatment can be varied widely in view of the advantageous properties of the present compounds. No mortality of test animals due the present compounds has been noted in studies employing dosage levels up to 20 mg/kg.

For both diagnosis and treatment, the instant compounds can be administered by the oral, intravenous or intramuscular routes. They can be formulated as lyophilized sterile, pyrogen-free compounds, preferably in the form of basic salts, e.g., sodium salt. The preferred dosage forms are provided as injectable solutions (isotonic).

The irradiation source used in treatment of tumors containing compounds of this invention is a filtered, high-intensity, continuous source or pumped dye, or other laser and light delivery system, which is capable of performing within the following limits: power intensity 20-500 mw/cm$^2$ at wavelengths between 620 and 760 nm. and a total output of at least 500 mw. or greater. Several currently commerical available lasers meet these criteria.

The present tetrapyrroles can be prepared by various synthetic methods which are found in the literature, e.g.,

Chlorin $e_6$

Willstatter, R. Stoll, A.; Investigations on Chlorophyll, (Trans., Schertz, F.M., Merz, A.R.,) p. 176. Science Printing Press, Lancaster, Pennsylvania, 1928.
Willstatter, R., Isler, M.; Ann. Chem., 390, 269 (1912).
Fisher, H., Baumler, R.; Ann Chem., 474, 65 (1929).
Fisher, H., Siebel, H.; Ann. Chem., 499, 84 (1932).
Conant, J.B., Mayer, W.W.; J. Amer. Chem. Soc., 52, 3013 (1930).
Fischer and Orth, "Des Chemie des Pyrrole" Akademische Verlazsgesellschaft, Leipzic, 1940, Vol. II, Part 2.

General Reference for Porphyrins

"Porphyrins and Metalloporphyrins" ed. Kevin M. Smith, Elsevier 1975 N.Y.

The therapeutic composition of the present invention can be administered to the host in a variety of forms adapted to the chosen route of administration, i.e., orally, intravenously, intramuscularly or subcutaneous routes.

The active compound may be oraly administered, for example, with an inert diluent or with an assimilable edible carrier, or it may be enclosed in hard or soft shell gelatin capsule, or it may be compressed into tablets, or it may be incorporated directly with the food fo the diet. For oral therapeutic administration, the active compound may be incorporated with excipients and used in the form of ingestible tablets, buccal tablets, troches, capsules, elexirs, suspensions, syrups, wafers, and the like. Such compositions and preparations should contain at least 0.1% of active compound. The percentage of the compositions and preparations may, of course, be varied and may conveniently be between 2 to 60% of the weight of the unit. The amount of active compound in such therapeutically useful compositions is such that a suitable dosage will be obtained. Preferred compositions or preparations according to the present invention are prepared so that an oral dosage unit form contains between 50 and 300 mg of active compound.

The tablets, troches, pills, capsules may also contain the following: A binder such as gum tragacanth, acacia, corn starch or gelatin; excipients such as dicalcium phosphate; a disintegrating agent such as corn starch, potato starch, alginic acid and the like; a lubricant such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials maly be present as coatings or to otherwise modify the physical form of the dosage unit. For instance, tablets, pills, or capsules may be coated with shellac, sugar or both. A syrup or elixir may contain the ative compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the

active compound may be incorporated into sustained-release preparations and formulations.

The active compound may also be administered parenterally or intraperitoneally. Solutions of the active compound as a free base or pharmacologically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol. phenol, sorbic acid and thimerosal. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The present new compounds may also be applied directly to tumors, whether internal or external, in the host in topical compositions. Exemplary compositions include solutions of the new compounds in solvents, particularly aqueous solvents, most preferably water. Alternatively, for topical application particularly to skin tumors, the present new compounds may be dispersed in the usual cream or salve-formulations commonly used for the purpose or may be provided in the form of spray solutions or suspensions which may include a propellant usually employed in aerosol preparations.

As used herein, "pharmaceutically acceptable carrier" includes any and all solvents, dispersion media, coatings, antibacterial and antifungal agents, isotonic and absorption delaying agents. The use of such media and agents for pharmaceutical active substances is well known in the art. Except insofar as any conventional media or agent is incompatable with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of tumors in living subjects.

The following examples further illustrate the invention:

EXAMPLE 1

Preparation chlorin $e_6$

Chlorin $e_6$ was prepared according to the procedure of Fischer and Stern, Di Chemie Des Pyrroles, Volume II, second half, Leipsig 1940, Akademische Verlagsgesellschaft, pp. 91-93.

EXAMPLE 2

Preparation of formylchlorin $e_6$(2-Desvinyl-2-Formyl-Chlorin $e_6$

500 mg of chlorin $e_6$ trimethyl ester was prepared according to the procedure of Fisher and Stern, in Di Chemie Des Pyrroles, Volume II, second half, Leipsig 1940, Akademische Verlagsgesellschaft, pp. 98-102. The chlorin $e_6$ trimethyl ester was dissoved in 600 ml of refluxing acetone. 400 mg of Potassium permanganate and 800 mg of magnesium sulfate dissolved in 130 ml of $H_2O$ were added slowly over approximately a one hour period to the refluxing acetone solution. The solution was allowed to reflux for 1/2 hour after addition was complete. After cooling, 300 ml of methylene chloride was added, and the mixture was washed 3 times with water in a separatory funnel. The volume of methylene chloride was reduced and the product chromatographed on silica gel, eluting with a gradually increasing percentage of ethyl acetate in the $CH_2Cl_2$. The first major brown band which eluted was collected as the product, 2-Desvinyl-2-Formyl-Chlorin $e_6$. Yield 94 mg.

The product was saponified by dissolution in refluxing n-propanol (0.1 ml/mg) and addition of 6 fold equivalent of 1N KOH. The tripotassium salt was filtered off, washed with n-propanol and dried under vacuum, forming 2-formyl chlorin $e_6$.

EXAMPLE 3

Preparation of Deuterochlorin $e_6$(2-desvinyl-chlorin $e_6$)

Deuterochlorin $e_6$

Deuterchlorin $e_6$ trimethyl ester was prepared according to the procedure in Fisher and Stern in Di Chemie Des Pyrroles, Volume II, second half, Leipsig 1940, Akademische Verlagsgesellschaft, p. 104. The trimethyl ester was then hydrolyzed to the free acid state by dissolution in refluxing n-propanol (0.1 ml/mg) and adding 6 fold equivalent amounts of 1N KOH. The product was collected by filtration, after cooling, as the potassium salt and dried under vaccum.

EXAMPLE 4

Preparation of acetyl-chlorin $e_6$(2-desvinyl-2-acetyl chlorin $e_6$)

2-acetyl chlorin $e_6$

2-acetyl chlorin $e_6$ trimethyl ester was prepared according to the procedure of Fischer and Stern, Di Chemie Des Pyrroles, Volume II, second half, Leipsig 1940, Akademische Verlagsgesellschaft, p. 185.
The trimethyl ester was then hydrolyzed to the free acid state by dissolution in refluxing n-propanol (0.1 ml/mg) and adding 6 fold equivalent amounts of 1N KOH. The product was collected by filtration, after cooling, as the potassium salt and dried under vaccum.

EXAMPLE 5

Preparation of Mesochlorin $e_6$

Mesochlorin $e_6$

Mesochlorin $e_6$ trimethyl ester was prepared according to the procedure of Fischer and Stern, Di Chemie Des Pyrroles, Volume II, second half, Leipsig 1940, Akademische Verlagsgesellschaft p. 102.

The mesochlorin $e_6$ trimethyl ester was then hydrolyzed to the free acid state by dissolution in refluxing n-propanol (0.1 ml/mg) and adding 6 fold equivalent amounts of 1N KOH. The product was collected by filtration, after cooling, as the potassium salt and dried under vacuum.

Physical characteristics of the compounds (relative polarity) is measured by a standard chromatographic system. The chromatographic data (Rf values) were measured on Baker silica gel-C18 thin layer chromatographic plates, the particle size of which is 20 $\mu$M, and the coating thickness of which is 200 $\mu$M. The solvent system for these chromatographic runs consisted of 75% methanol, and 25% 0.01 M potassium. phosphate buffer, pH 6.85. The compounds were spotted and dried on the plate as the sodium

salts, at approximately neutral pH and minimum salt concentrations. The Rf values for the various derivatives are tabulated in TABLE 1. Spectroscopic data are indicated in TABLE 2.

TABLE 1

| Rf VALUES | | |
|---|---|---|
| Compounds | Derivative | Rf |
| Chlorin $e_6$ | ---- | 0.66 |
| 2-formyl chlorin $e_6$ | ----- | 0.74 |
| 2-acetyl chlorin $e_6$ | ----- | 0.71 |
| Deurero chlorin $e_6$ | ----- | 0.79 |
| Mesochlorin $e_6$ | ----- | 0.69 |

**TABLE II**

**Spectroscopic Absorption Data**

Solvent in all cases is p-dioxane.

| Compounds | Absorption Maxima (nm) in Visible Region | Soret Band nM |
|---|---|---|
| Mesochlorin $e_6$ | 651 | 399 |
| 2-acetyl-chlorin $e_6$ | 712,683 | 410 |
| 2-formyl-chlorin $e_6$ | 687 | 412 |
| Deuterochlorin $e_6$ | 653 | 398 |
| Chlorin $e_6$ | 666 | 402 |

EXAMPLE 6

The phototdynamic therapy experiments have been carried out on Buffalo rats, using the transplantable tumor, Morris Hepatoma 7777. The tumors were transplanted subcutaneously on the outside of the thigh. During treatment, the tumors ranged in size between 1 and 2.5 cm in diameter.

The general treatment regime is as follows. The rats are injected with a solution of the chlorin prepared as follows: 20mg of the sodium salt of the chlorin was dissolved in 1 ml of 0.9% NaCl. The chlorin solution was then injected intravenously through the external jugular while the rat was anesthetized with ether. The volume of solution injected was calculated based upon the weight of the animal and the dosage, on a weight to weight basis, for the particular experiment. A specified time interval was then allowed to elapse before light treatment was instigated.

Light treatment of the rats was without anesthesia. The rats were restrained, and hair removed in the treatment area and treated with laser light from a Cooper Aurora argon pumped, tunable dye laser.

The laser was equipped with a fiber optic light delivery system coupled to a microlens system developed by Dr. Daniel Doiron, D.R.D. Consulting, Santa Barbara, California.

The lens disperses the laser beam, providing a circular distribution of light with homogenous light intensity throughout the area of the incident light beam. The wavelength of light was adjusted using a Hartridge reversion spectroscope. The light intensity was determined using a Yellow Springs Instrument, Model 65A, radiometer.

The micro lens was positioned at such a distance from the skin of the animal so as to provide an illumination diameter of 1.5cm, and the light flux was varied by control of the laser output.

Subsequent to illumination, the animal was returned to its cage and, 24 hours later, it was treated intravenously in the external jugular vein with 14 mg of Evans Blue dye, dissolved in 250 µl of 0.9% NaCl. Two hours after injection, the rat was sacrificed and the tumor cross-sectioned. The extent of tumor necrosis was assessed by the lack of dye uptake, and the depth of the necrotic cross section of the tumor was recorded in millimeters.

The following summary of the experimental results includes a range of wavelengths, dosages, intensities, and time intervals for treatment. This has been necessary, in order to attempt to establish the optimal conditions for phototherapy utilizing this new drug. The optimum conditions may yet be determined, but the conditions described result in measurable and significant damage to the tumors.

In all cases except where noted, tissue damage occurred selectively to the tumor tissue as assayed by the Evans Blue method, even though, in nearly all cases, normal skin overlayed the tumor and the treatment area overlapped significant areas of normal muscle tissue.

The photodynamic therapy data is present in tabular form. Column No. 2 is the total light dose administered in terms of Joules per square centimeter. Column No. 3 is the dose of chlorin administered in terms of mg of drug per kilogram of rat body weight. Column No. 4 is the time lapse between administration of drug and treatment with laser light. Column No.5 is the wavelength of treatment light in nanometers. Column No. 6 is the intensity of the treatment light in milliwatts per square centimeter. In Column No. 7, $\bar{x}$ is the mean depth of necrosis in millimeters of the tumor tissue, i.e. the distance from the necrotic tip of the tumor next to the skin to the necrotic edge of the tumor most distant from the skin.

S.D. is the standard deviation of $\bar{x}$.

(N) is the number of tumors or legs involved in the experiment.

Column No. 8 is the range of depth of necrosis in millimeters within the group.

Chlorin $e_6$ was administered in accordance with the foregoing procedure and the results are summarized below:

| Tumor | Joules/ $cm^2$ | Drug dose mg/kg | Time in hrs btwn drugs & light | Wave lnth nm | Intensity $mW/cm^2$ | $\bar{x}$ s.d. (n) | Range mm |
|---|---|---|---|---|---|---|---|
| 7777 | 20 | 20 | 24 | 665 | 100 | 4.5±0.9 (3) | 3.5-5 |

Using the foregoing procedure, similar results are observed using: mesochlorin $e_6$, bacteriochlorin $e_6$, 2-desvinylchlorin $e_6$, mesochlorin $e_6$, 2-acetylchlorin $e_6$, 2-formylchlorin $e_6$, and rhodin $g_7$.

## EXAMPLE 7

Male Buffalo rats (approximately 200g each), with transplanted subcutaneous Morris Hepatoma 7777 tumors on the exterior portion of the hind legs were injected intravenously via the exterior jugular with chlorin $e_6$ or Photofrin II, [(2)]at a dosage of 20mg/kg. Twenty four hours later, the areas over the tumors were shaved and the light treatment was begun.

Light from a Cooper Aurora argon pumped tunable dye laser was administered via a micro lens system (developed by Dr. Daniel Doiron, D.R.D. Consulting, Santa Barbara, California) coupled through a quartz fiber to the laser. The optical properties of the lens are such that the light exits the lens in a circular pattern with a homogenous intensity throughout the lighted area. The diameter of the lighted area is a function of its distance from the lens.

The light intensity was measured with a Yellow Springs Instrument Model 65A Radiometer at the point of treatment. A 1.5 cm diameter circle of the animal's skin, centered as closely as possible over the tumor, was irradiated in all the experiments. In all cases light at an intensity of 100 $mW/cm^2$ was administered until

(1) M.C. Berenbaum, Br. J. Cancer 45:571(1982)

(2) Photofrin II is a trade name for purified Hematoporphyrin Derivative (HPD). It was obtained from Oncology Research and Development in Roswell Park, Buffalo, New York.

a total dosage of 20 joules/$cm^2$ had been achieved. Chlorin $e_6$ rats were treated with 665 nanometer light in order to approximately match its peak of absorption in the red region of the spectrum. Photofrin II rats were treated with 630 nanometer light in accordance with the preponderance of photodynamic therapy experiments described in the literature, using this drug, and its forerunner HPD or Photofrin I. The wavelengths of light were determined with the aid of a Hartridge reversion spectroscope to within one nanometer.

Twenty four hours after light treatment, each rat received 14mg of Evans Blue dye intravenously. [(1)]After an additional two hours, the rats were sacrificed and the tumors were sectioned vertically through the center of the light treated area.

Unaffected tumor was stained blue, as was normal tissue. Necrotic areas were white or red in appearance. Measurements on both the whole tumors and necrotic areas of the tumors were made vertically and horizontally with calipers to the nearest one half millimeter.

Nineteen tumors here evaluated for Chlorin $e_6$ and light effect while twenty two tumors were assessed for Photofrin II and light effect.

The following data were obtained for Chlorin $e_6$

The number of tumors treated was 19
The number of tumors developing necrosis following treatment was 17

| | Mean | S.D. |
|---|---|---|
| The mean width of tumor necrosis was | 12.2 ± | 4.7 mm |
| The mean depth of tumor necrosis was | 4.6 ± | 1.4 mm |

Tumor sizes for the treatment group :

| | Mean | S.D. |
|---|---|---|
| The width of tumor cross section | 13.4 ± | 5.7 mm |
| The depth of tumor cross section | 7.8 ± | 2.7 mm |

The following data were obtained for Photofrin II

The number of tumors treated was 22
The number of tumors developing necrosis following treatment was 9.

| | Mean | S.D. |
|---|---|---|
| The mean width of tumor necrosis was | 4.5 ± | 3.2 mm |
| The mean depth of tumor necrosis was | 2.4 ± | 1.0 mm |

| Tumor sizes for the treatment group: | Mean | S.D. |
|---|---|---|
| The width of tumor cross section | 17.0 ± | 6.4 mm |
| The depth of tumor cross section | 9.2 ± | 3.3 mm |

EXAMPLE 8

The treatment and evaluation procedure is as follows: DBA/2 Ha Ros-d+Ha mice with SmT-F transplanted tumors either in the exterior part of the hind leg or the side of the mouse were injected intravenously via the external jugular or intraperitoneally with the photosensitizing drug. At the specified time

(1) M.C. Berenbaum. Br, J, Cancer. 45:571 (1982).

after injections the area over the tumor was shaved and the light treatment begun.

Light from a Cooper Aurora argon pumped tunable dye laser was administered via a micro lens system (developed by Dr. Daniel Doiron, D.R.D. Consulting, Santa Barbara, California) coupled through a quartz fiber to the laser. The optical properties of the lens are such that the light exits the lens in a circular pattern with homogenous intensity throughout the lighted area. The diameter of the lighted area is a function of the distance from the lens,

The light intensity was measured with a Yellow Springs Instrument Model 65 A Radiometer at the point of treatment. A 1.5 cm diameter circle of the animal's skin, centered as closely as possible over the tumor, was irradiated in all the experiments. The intensity, wavelength, and dosage of of light is included in the data for individual groups of animals. Wavelengths are adjusted, using a Hartridge reversion spectroscope to within 1 nm of the stated value.

Twenty, four hours after light treatment, each mouse received 5 mg of Evans Blue Dye intravenously [(1)]. After an additional two hours, the mice were sacrificed and the tumors were sectioned vertically through the center of the light treated area. Unaffected tumor was stained blue as was unaffected normal tissue. Necrotic or affected areas were white or red in appearance. Measurements on both the whole tumors and affected areas of the tumors were made vertically and horizontally with calipers to the nearest one half millimeter.

(1) M.C. Berenbaum. Br, J, Cancer. 45:571 (1982).

TABLE 3 - MOUSE DATA

| COMPOUND USED | 2-acetyl chlorin e6 | | | |
|---|---|---|---|---|
| ANIMAL GROUP NO. | 74 | 74 | 74 | 74 |
| DATE EXPERIMENT STARTED | -- | -- | -- | -- |
| MOUSE NO. | 3 | 2 | 1 | 4 |
| SEX OF MOUSE | m | m | m | m |
| WT. OF MOUSE (gms) | 22.8 | 24.7 | 24.8 | 22.6 |
| DRUG DOSE (mg/kg) | 100.0 | 100.0 | 100.0 | 100.0 |
| METHOD OF DRUG INTRODUCTION | iv | iv | iv | iv |
| TIME BETW. DRUG INTRODUCTION + LIGHT TREATMENT (hrs) | 24.0 | 24.0 | 24.0 | 24.0 |
| TUMOR TYPE | SMT-F | SMT-F | SMT-F | SMT-F |
| POSITION OF TUMOR ON ANIMAL | r leg | r leg | r leg | r leg |
| LIGHT TREATMENT INTENSITY ($mW/cm^2$) | 200.0 | 200.0 | 200.0 | 200.0 |
| LIGHT DOSE ($J/cm^2$) | 300.0 | 300.0 | 300.0 | 300.0 |
| WAVE LENGTH USED TO TREAT TUMOR (nm) | 680 | 680 | 680 | 680 |
| DATE ANIMAL INJECTED WITH DRUG | -- | -- | -- | -- |
| LENGTH OF TUMOR ON INJECTION DATE (cm) | 1.10 | 1.15 | 1.10 | 1.70 |
| WIDTH OF TUMOR ON INJECTION DATE (cm) | 0.70 | 0.70 | 0.65 | 0.70 |
| DEPTH OF TUMOR ON INJECTION DATE (cm) | 0.45 | 0.60 | 0.65 | 0.50 |
| DATE ANIMAL SACRIFICED | -- | -- | -- | -- |
| LENGTH OF TUMOR ON SACRIFICE DATE (cm) | 0.70 | 1.20 | 1.10 | 1.55 |
| WIDTH OF TUMOR ON SACRIFICE DATE (cm) | 0.55 | 0.80 | 0.60 | 1.20 |
| DEPTH OF TUMOR ON SACRIFICE DATE (cm) | 0.50 | 0.55 | 0.50 | 0.60 |
| LENGTH OF EFFECT UPON TUMOR ON SACRIFICE DATE (cm) | 0.55 | 0.00 | 0.00 | 0.00 |
| WIDTH OF EFFECT UPON TUMOR ON SACRIFICE DATE (cm) | 0.55 | 0.00 | 0.00 | 0.00 |
| DEPTH OF EFFECT UPON TUMOR ON SACRIFICE DATE (cm) | 0.15 | 0.00 | 0.00 | 0.00 |
| COMMENTS AS RESULT OF TUMOR ASSESSMENT | | no effect | no effect | no effect |

TABLE 4 - MOUSE DATA

| COMPOUND USED | 2-formyl chlorin e6 | | | | |
|---|---|---|---|---|---|
| ANIMAL GROUP NO. | 71 | 71 | 71 | 71 | 71 |
| DATE EXPERIMENT STARTED | — | — | — | — | — |
| MOUSE NO. | 1 | 2 | 3 | 4 | 5 |
| SEX OF MOUSE | f | f | f | f | f |
| WT. OF MOUSE (gms) | 20.0 | 20.8 | 20.4 | 17.6 | 20.6 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY (mW/cm$^2$) | 200.0 | 200.0 | 200.0 | 200.0 | 200.0 |
| LIGHT DOSE (J/cm$^2$) | 300.0 | 300.0 | 300.0 | 300.0 | 300.0 |
| WAVE LENGTH USED TO TREAT TUMOR (nm) | 690 | 690 | 690 | 690 | 690 |
| DATE ANIMAL INJECTED WITH DRUG | — | — | — | — | — |
| LENGTH OF TUMOR ON INJECTION DATE (cm) | 0.70 | 0.95 | 0.80 | 1.10 | 1.30 |
| WIDTH OF TUMOR ON INJECTION DATE (cm) | 0.25 | 0.60 | 0.50 | 0.70 | 0.80 |
| DEPTH OF TUMOR ON INJECTION DATE (cm) | 0.20 | 0.40 | 0.20 | 0.30 | 0.60 |
| DATE ANIMAL SACRIFICED | — | — | — | — | — |
| LENGTH OF TUMOR ON SACRIFICE DATE (cm) | 0.55 | 1.10 | 0.95 | 1.30 | 0.00 |
| WIDTH OF TUMOR ON SACRIFICE DATE (cm) | 0.45 | 0.95 | 0.60 | 1.00 | 0.00 |
| DEPTH OF TUMOR ON SACRIFICE DATE (cm) | 0.20 | 0.45 | 0.40 | 0.50 | 0.00 |
| LENGTH OF EFFECT UPON TUMOR ON SACRIFICE DATE (cm) | 0.55 | 1.10 | 0.95 | 1.30 | 0.00 |
| WIDTH OF EFFECT UPON TUMOR ON SACRIFICE DATE (cm) | 0.45 | 0.95 | 0.60 | 1.00 | 0.00 |
| DEPTH OF EFFECT UPON TUMOR ON SACRIFICE DATE (cm) | 0.20 | 0.40 | 0.25 | 0.45 | 0.00 |
| COMMENTS AS RESULT OF TUMOR ASSESSMENT | total kill | total kill skin damage 0.7 x 0.7 cm | total kill brown skin 0.7 x 0.65 cm | brown skin 1.0 x 1.0 cm | died after ½ light treatment |

TABLE 5 - MOUSE DATA

| COMPOUND USED | chlorin e6 | | | | |
|---|---|---|---|---|---|
| ANIMAL GROUP NO. | 69 | 69 | 69 | 69 | 69 |
| DATE EXPERIMENT STARTED | — | — | — | — | — |
| MOUSE NO. | 1 | 2 | 3 | 4 | 5 |
| SEX OF MOUSE | f | f | f | f | f |
| WT. OF MOUSE (gms) | 23.8 | 21.2 | 21.8 | 22.5 | 21.9 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 200.0 | 200.0 | 200.0 | 200.0 | 200.0 |
| LIGHT DOSE ($J/cm^2$) | 300.0 | 300.0 | 300.0 | 300.0 | 300.0 |
| WAVE LENGTH USED TO TREAT TUMOR (nm) | 665 | 665 | 665 | 665 | 665 |
| DATE ANIMAL INJECTED WITH DRUG | — | — | — | — | — |
| LENGTH OF TUMOR ON INJECTION DATE (cm) | 0.65 | 0.60 | 1.00 | 0.50 | 1.10 |
| WIDTH OF TUMOR ON INJECTION DATE (cm) | 0.65 | 0.60 | 0.85 | 0.60 | 0.85 |
| DEPTH OF TUMOR ON INJECTION DATE (cm) | 0.30 | 0.25 | 0.45 | 0.40 | 0.40 |
| DATE ANIMAL SACRIFICED | — | — | — | — | — |
| LENGTH OF TUMOR ON SACRIFICE DATE (cm) | 1.40 | 1.30 | 1.10 | 0.40 | 1.15 |
| WIDTH OF TUMOR ON SACRIFICE DATE (cm) | 1.00 | 0.95 | 0.90 | 0.40 | 1.00 |
| DEPTH OF TUMOR ON SACRIFICE DATE (cm) | 0.55 | 0.45 | 0.50 | 0.20 | 0.50 |
| LENGTH OF EFFECT UPON TUMOR ON SACRIFICE DATE (cm) | 1.00 | 0.70 | 0.60 | 0.40 | 0.80 |
| WIDTH OF EFFECT UPON TUMOR ON SACRIFICE DATE (cm) | 0.90 | 1.00 | 0.40 | 0.40 | 0.50 |
| DEPTH OF EFFECT UPON TUMOR ON SACRIFICE DATE (cm) | 0.35 | 0.30 | 0.20 | 0.30 | 0.20 |
| COMMENTS AS RESULT OF TUMOR ASSESSMENT | brown skin 1.9 x 1.1 cm | brown skin 0.65 x 0.65 cm patchy effect | brown skin 0.55 x 0.5 cm trouble injecting | brown skin 0.70 x 0.65 cm | brown skin 0.8 x 0.75 cm |

TABLE 5 - MOUSE DATA (cont.)

| COMPOUND USED | chlorin e6 | | | | |
|---|---|---|---|---|---|
| ANIMAL GROUP NO. | 69 | 69 | 69 | 69 | 69 |
| DATE EXPERIMENT STARTED | -- | -- | -- | -- | -- |
| MOUSE NO. | 6 | 7 | 8 | 9 | 10 |
| SEX OF MOUSE | f | f | f | f | f |
| WT. OF MOUSE (gms) | 21.0 | 21.4 | 22.0 | 22.0 | 19.3 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | rleg | r leg |
| LIGHT TREATMENT INTENSITY (mW/cm$^2$) | 200.0 | 200.0 | 200.0 | 200.0 | 200.0 |
| LIGHT DOSE (J/cm$^2$) | 300.0 | 300.0 | 300.0 | 300.0 | 300.0 |
| WAVE LENGTH USED TO TREAT TUMOR (nm) | 665 | 665 | 665 | 665 | 665 |
| DATE ANIMAL INJECTED WITH DRUG | -- | -- | -- | -- | -- |
| LENGTH OF TUMOR ON INJECTION DATE (cm) | 0.85 | 0.90 | 0.70 | 0.75 | 0.95 |
| WIDTH OF TUMOR ON INJECTION DATE (cm) | 0.70 | 0.95 | 0.50 | 0.55 | 0.70 |
| DEPTH OF TUMOR ON INJECTION DATE (cm) | 0.35 | 0.45 | 0.30 | 0.50 | 0.40 |
| DATE ANIMAL SACRIFICED | -- | -- | -- | -- | -- |
| LENGTH OF TUMOR ON SACRIFICE DATE (cm) | 1.10 | 1.40 | 0.70 | 1.05 | 1.10 |
| WIDTH OF TUMOR ON SACRIFICE DATE (cm) | 0.90 | 1.25 | 0.60 | 0.90 | 0.80 |
| DEPTH OF TUMOR ON SACRIFICE DATE (cm) | 0.45 | 0.65 | 0.45 | 0.50 | 0.50 |
| LENGTH OF EFFECT UPON TUMOR ON SACRIFICE DATE (cm) | 0.65 | 0.85 | 0.70 | 0.95 | 0.50 |
| WIDTH OF EFFECT UPON TUMOR ON SACRIFICE DATE (cm) | 0.65 | 0.85 | 0.50 | 0.50 | 0.80 |
| DEPTH OF EFFECT UPON TUMOR ON SACRIFICE DATE (cm) | 0.25 | 0.20 | 0.25 | 0.30 | 0.25 |
| COMMENTS AS RESULT OF TUMOR ASSESSMENT | brown skin 0.60 x 0.65 cm | | brown skin 0.7 x 0.7 cm muscle damage 0.6 x 0.2 cm | brown skin 0.55 x 0.55 cm total kill, muscle damage | brown skin 0.8 x 0.8 cm |

TABLE 6 - MOUSE DATA

| COMPOUND USED | Deuterochlorin e6 | | | | |
|---|---|---|---|---|---|
| ANIMAL GROUP NO. | 77 | 77 | 77 | 77 | 77 |
| DATE EXPERIMENT STARTED | — | — | — | — | — |
| MOUSE NO. | 1 | 2 | 3 | 4 | 5 |
| SEX OF MOUSE | m | m | m | m | m |
| WT. OF MOUSE (gms) | 19.4 | 20.2 | 18.8 | 23.6 | 19.9 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY (mW/cm$^2$) | 200.0 | 200.0 | 200.0 | 200.0 | 200.0 |
| LIGHT DOSE (J/cm$^2$) | 300.0 | 300.0 | 300.0 | 300.0 | 300.0 |
| WAVE LENGTH USED TO TREAT TUMOR (nm) | 655 | 655 | 655 | 655 | 655 |
| DATE ANIMAL INJECTED WITH DRUG | — | — | — | — | — |
| LENGTH OF TUMOR ON INJECTION DATE (cm) | 0.90 | 1.15 | 0.85 | 1.05 | 1.15 |
| WIDTH OF TUMOR ON INJECTION DATE (cm) | 0.60 | 0.60 | 0.60 | 0.45 | 0.50 |
| DEPTH OF TUMOR ON INJECTION DATE (cm) | 0.45 | 0.40 | 0.45 | 0.30 | 0.30 |
| DATE ANIMAL SACRIFICED | — | — | — | — | — |
| LENGTH OF TUMOR ON SACRIFICE DATE (cm) | 1.20 | 1.15 | 1.10 | 0.70 | 0.90 |
| WIDTH OF TUMOR ON SACRIFICE DATE (cm) | 0.80 | 1.15 | 0.70 | 0.60 | 0.60 |
| DEPTH OF TUMOR ON SACRIFICE DATE (cm) | 0.50 | 0.60 | 0.50 | 0.35 | 0.30 |
| LENGTH OF EFFECT UPON TUMOR ON SACRIFICE DATE (cm) | 1.30 | 1.15 | 1.10 | 1.50 | 1.20 |
| WIDTH OF EFFECT UPON TUMOR ON SACRIFICE DATE (cm) | 0.80 | 1.15 | 0.70 | 0.75 | 0.70 |
| DEPTH OF EFFECT UPON TUMOR ON SACRIFICE DATE (cm) | 0.55 | 0.60 | 0.50 | 0.60 | 0.70 |
| COMMENTS AS RESULT OF TUMOR ASSESSMENT | red area on skin 0.75 x 0.65 cm extensive muscle damage | total kill red skin 1.0 x 0.8 cm surface muscle damage | red skin 0.75 x 0.75 cm total kill extensive muscle damage | red skin 1.0 x 0.8 cm very extensive muscle damage | red skin 1.0 x 0.8 cm extensive muscle damage |

TABLE 7 - MOUSE DATA

| COMPOUND USED | Mesochlorin e6 | | | | |
|---|---|---|---|---|---|
| ANIMAL GROUP NO. | 76 | 76 | 76 | 76 | 76 |
| DATE EXPERIMENT STARTED | — | — | — | — | — |
| MOUSE NO. | 1 | 2 | 3 | 4 | 5 |
| SEX OF MOUSE | m | m | m | m | m |
| WT. OF MOUSE (gms) | 19.4 | 21.3 | 20.1 | 24.1 | 19.2 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 200.0 | 200.0 | 200.0 | 200.0 | 200.0 |
| LIGHT DOSE ($J/cm^2$) | 300.0 | 300.0 | 300.0 | 300.0 | 300.0 |
| WAVE LENGTH USED TO TREAT TUMOR (nm) | 652 | 652 | 652 | 652 | 652 |
| DATE ANIMAL INJECTED WITH DRUG | — | — | — | — | — |
| LENGTH OF TUMOR ON INJECTION DATE (cm) | 0.45 | 1.25 | 0.95 | 1.10 | 1.00 |
| WIDTH OF TUMOR ON INJECTION DATE (cm) | 0.45 | 0.60 | 0.55 | 0.60 | 0.55 |
| DEPTH OF TUMOR ON INJECTION DATE (cm) | 0.25 | 0.40 | 0.35 | 0.25 | 0.30 |
| DATE ANIMAL SACRIFICED | — | — | — | — | — |
| LENGTH OF TUMOR ON SACRIFICE DATE (cm) | 0.35 | 0.60 | 0.95 | 1.05 | 0.95 |
| WIDTH OF TUMOR ON SACRIFICE DATE (cm) | 0.35 | 0.55 | 0.65 | 0.55 | 0.65 |
| DEPTH OF TUMOR ON SACRIFICE DATE (cm) | 0.15 | 0.40 | 0.50 | 0.40 | 0.55 |
| LENGTH OF EFFECT UPON TUMOR ON SACRIFICE DATE (cm) | 0.35 | 0.00 | 0.10 | 0.10 | 0.00 |
| WIDTH OF EFFECT UPON TUMOR ON SACRIFICE DATE (cm) | 0.35 | 0.00 | 0.10 | 0.10 | 0.00 |
| DEPTH OF EFFECT UPON TUMOR ON SACRIFICE DATE (cm) | 0.15 | 0.00 | 0.10 | 0.10 | 0.00 |
| COMMENTS AS RESULT OF TUMOR ASSESSMENT | total kill | no effect | thought it was infection | | no effect |

The results of Table 3 - Table 7 are summarized in Table 8.

TABLE 8

| compound | drug dose mg/kg | method of injection | time in hrs between drug dose & light | tumor type | light intensity mW/cm2 | light dose, J/cm$^2$ | wave-length nm | (n) | $\bar{X}$ ± s.d. (cm) | range cm |
|---|---|---|---|---|---|---|---|---|---|---|
| 2-acetyl chlorin e6 | 100 | iv | 24 | SMT-F | 200 | 300 | 680 | (4) | 0.04±0.08 | 0.00-0.15 |
| 2-formyl chlorin e6 | 100 | iv | 24 | SMT-F | 200 | 300 | 690 | (4) | 0.33±0.12 | 0.20-0.45 |
| chlorin e6 | 100 | iv | 24 | SMT-F | 200 | 300 | 665 | (10) | 0.26±0.05 | 0.20-0.35 |
| deuterochlorin e6 | 100 | iv | 24 | SMT-F | 200 | 300 | 655 | (5) | 0.59±0.07 | 0.50-0.70 |
| mesochlorin e6 | 100 | iv | 24 | SMT-F | 200 | 300 | 652 | (4) | 0.06±0.08 | 0.00-0.15 |

The preparation of pharmacological dosages for the administration of the active ingredient, that is the amino acid porphyrin adducts, which were prepared in Examples 1-8 hereinabove, is as follows:

EXAMPLE 9

A tablet base was prepared by blending the following ingredient in the proportion by weight indicated:

| | Grams |
|---|---|
| Sucrose, USP | 80.3 |
| Tapioca Starch | 13.2 |
| Magnesium Stearate | 4.4 |

Into this base, there was blended sufficient porphyrin to provide tablets each containing 100 mg of active ingredient.

EXAMPLE 10

A blend was prepared containing the following ingredients:

| | |
|---|---|
| Calcium phosphate | 17.6 |
| Dicalcium phosphate | 18.8 |
| Magnesium trisilicate, USP | 5.2 |
| Lactose, U.S.P. | 5.2 |
| Potato Starch | 5.2 |
| Magnesium Stearate A | 0.8 |
| Magnesium Stearate B | 0.32 |
| Porphyrin Adducts | 20 |

This blend was divided and formed into capsules each containing 25 mg of active ingredient.

EXAMPLE 11

To a commercially available raspberry flavored sugar syrup is added the equivalent of 40 mg of the porphyrin adduct per milliliter and the mixture is homogenized in a mechanical device for this purpose. This mixture is especially suitable for oral administration containing 200 mg of the active ingredient.

EXAMPLE 12

A sterile solution of the following composition is prepared: 200 mg of the sodium salt of the porphyrin adduct is dissolved in a 0.9% NaCl solution so that the final concentration is 20 mg/ml.

This solution is suitable for I.V. and I.M. administration.

EXAMPLE 13

The sodium salt of the porphyrin adduct is dissolved in 0.9% NaCl solution so that the final concentration is 5 mg/ml. This is placed in an aerosal dispenser with a hydrocarbon propellant. This preparation is suitable for topical application.

EXAMPLE 14

PREPARATION OF A METAL SALT

The sodium salt of the porphyrin adduct is prepared by dissolving said adduct in water containing an equimolar amount of sodium hydroxide and freeze drying the resulting mixture.

In this fashion, other metal salts are prepared including potassium, calcium, and lithium salts.

PREPARATION OF AN ACID SALT

The porphyrin adduct described in the preceding examples are converted to acid salts, e.g., hydrochloride, by dissolving in an aqueous solution containing an equivalent amount of acid, e.g., hydrochloric acid, and the solution is evaporated to dryness to obtain the solid salt. Alternately, alcoholic solutions of hydrogen chloride gas, dissolved in ethanol can be used in lieu of the aqueous acid solution and the acid salt is obtained by evaporation of the solvent or crystallization from the alcohol, e.g., by addition of a non-solvent.

**Claims**

1. A diagnostic composition for detection of mammalian tumors which comprises at least one fluorescent compound of the formula:

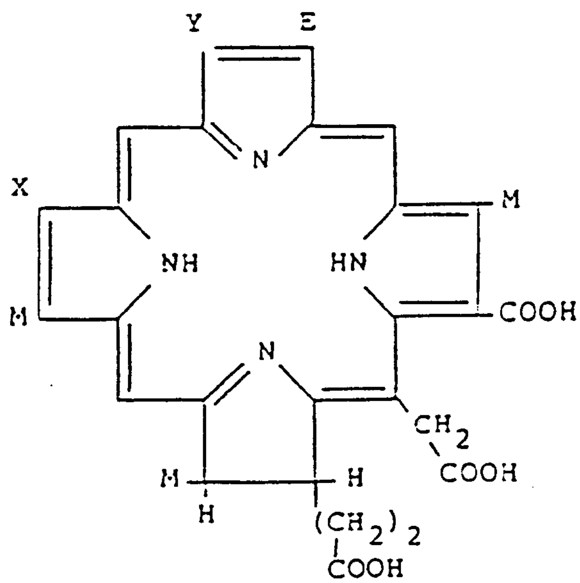

wherein:
X = H, vinyl, ethyl, acetyl or formyl;
Y = methyl or formyl;
M = methyl;
E = ethyl;
and pharmaceutically-acceptable salts thereof.

2. A therapeutic composition for the treatment of mammalian tumors which comprises an anti-tumor effective amount of at least one fluorescent compound of the formula:

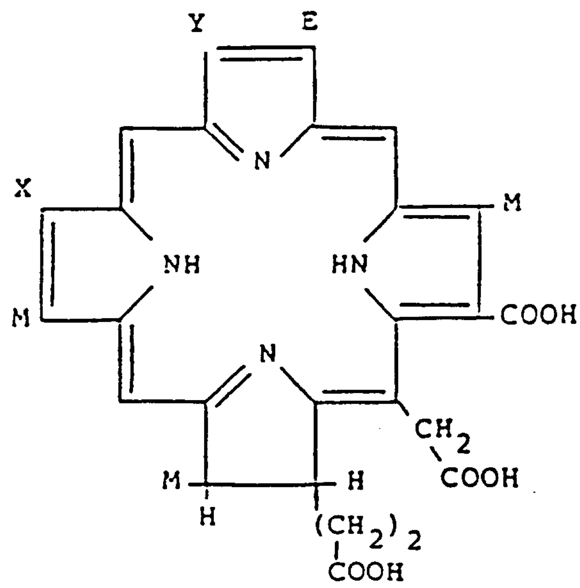

wherein:

X = H, vinyl, ethyl, acetyl, formyl;
Y = methyl or formyl;
M = methyl;
E = ethyl;

and pharmaceutically-acceptable salts thereof, with the proviso that said compound is not chlorin $\underline{e}_6$.

3. A therapeutic composition according to Claim 1, wherein said compound is chlorin $\underline{e}_6$, mesochlorin $\underline{e}_6$, bacteriochlorin $\underline{e}_6$, 2-desvinylchlorin $\underline{e}_6$, mesochlorin $\underline{e}_6$, 2-acetylchlorin $\underline{e}_6$, 2-formylchlorin $\underline{e}_6$ or rhodin $\underline{g}_7$.

4. The composition of Claim 1, wherein said compound can be activated by light having a wavelength of about 360 to 760 nm.

5. The therapeutic composition according to Claim 2, wherein said compound is mesochlorin $\underline{e}_6$, bacteriochlorin $\underline{e}_6$, 2-desvinylchlorin $\underline{e}_6$, mesochlorin $\underline{e}_6$, 2-acetylchlorin $\underline{e}_6$, 2-formylchlorin $\underline{e}_6$, or rhodin $\underline{g}_7$.

6. The composition of Claim 2, wherein said compound can be activated by light having a wavelength of about 620 to 760 nm.

**Patentansprüche**

1. Diagnostische Zusammensetzung zur Feststellung von Tumoren bei Säugetieren, welche mindestens eine fluoreszierende Verbindung der Formel

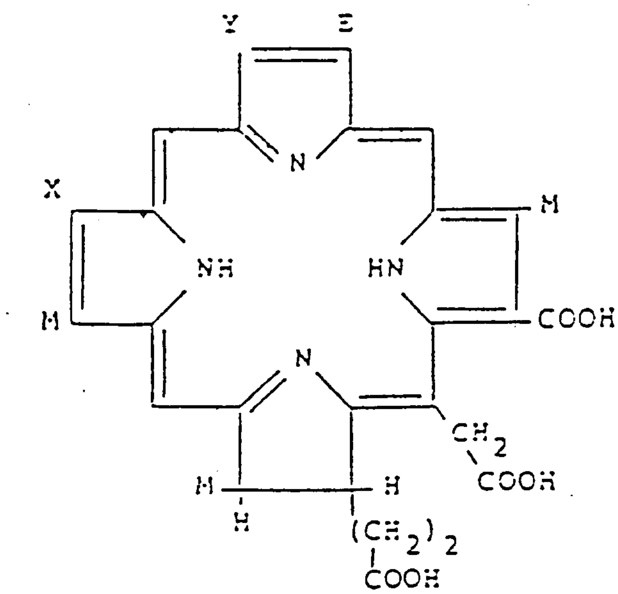

worin
X = H, Vinyl, Ethyl, Acetyl oder Formyl;
Y = Methyl oder Formyl;
M = Methyl;
E = Ethyl;
sowie pharmazeutisch akzeptable Salze davon umfaßt.

2. Therapeutische Zusammensetzung zur Behandlung von Tumoren bei Säugetieren, welche eine gegen Tumoren wirksame Menge mindestens einer fluoreszierenden Verbindung der Formel

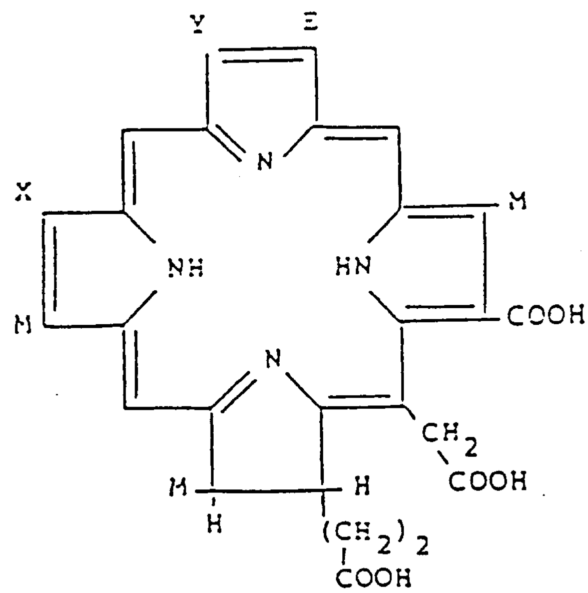

worin
X = H, Vinyl, Ethyl, Acetyl oder Formyl;
Y = Methyl oder Formyl;
M = Methyl;
E = Ethyl;
sowie pharmazeutisch akzeptable Salze davon umfaßt, unter der Voraussetzung, daß die genannte

Verbindung nicht Chlorin $e_6$ ist.

**3.** Therapeutische Zusammensetzung nach Anspruch 1, worin die genannte Verbindung Chlorin $e_6$, Mesochlorin $e_6$, Bacteriochlorin $e_6$, 2-Desvinylchlorin $e_6$, 2-Acetylchlorin $e_6$, 2-Formylchlorin $e_6$ oder Rhodin $g_7$ ist.

**4.** Zusammensetzung nach Anspruch 1, wobei die genannte Verbindung durch Licht einer Wellenlänge zwischen etwa 360 und 760 nm aktiviert werden kann.

**5.** Therapeutische Zusammensetzung nach Anspruch 2, worin die genannte Verbindung Mesochlorin $e_6$, Bacteriochlorin $e_6$, 2-Desvinylchlorin $e_6$, 2-Acetylchlorin $e_6$, 2-Formylchlorin $e_6$ oder Rhodin $g_7$ ist.

**6.** Zusammensetzung nach Anspruch 2, wobei die genannte Verbindung durch Licht einer Wellenlänge zwischen 620 und 760 nm aktiviert werden kann.

**Revendications**

**1.** Composition de diagnostic pour la détection de tumeurs chez les mammifères, qui comprend au moins un composé fluorescent de la formule :

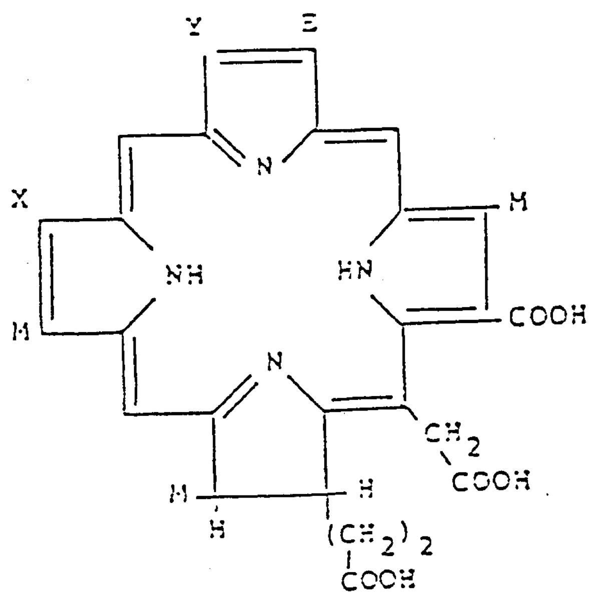

dans laquelle :
- X = H, vinyle, éthyle, acétyle, formyle ;
- Y = méthyle ou formyle ;
- M = méthyle ;
- E = éthyle ;

et ses sels pharmaceutiquement acceptables.

**2.** Composition thérapeutique pour le traitement de tumeurs chez les mammifères, qui comprend une quantité efficace pour lutter contre les tumeurs d'au moins un composé fluorescent de la formule :

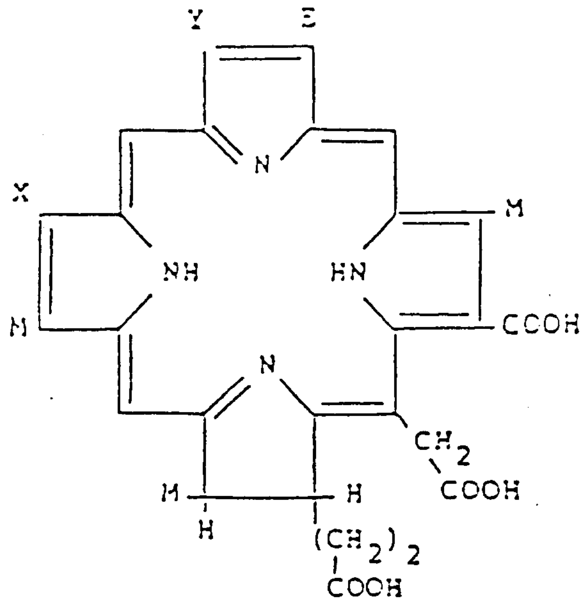

dans laquelle :

- X = H, vinyle, éthyle, acétyle, formyle ;
- Y = méthyle ou formyle ;
- M = méthyle ;
- E = éthyle ;

et ses sels pharmaceutiquement acceptables, à la condition que ledit composé ne soit pas la chlorine $e_6$.

**3.** Composition thérapeutique selon la revendication 1, dans laquelle ledit composé est la chlorine $e_6$, la mésochlorine $e_6$, la bactériochlorine $e_6$, la desvinyl-2 chlorine $e_6$, la mésochlorine $e_6$, l'acétyl-2 chlorine $e_6$, la formyl-2 chlorine $e_6$, ou la rhodine $g_7$.

**4.** Composition de la revendication 1, dans laquelle ledit composé peut être activé par une lumière ayant une longueur d'onde d'environ 360 à 760 nm.

**5.** Composition thérapeutique selon la revendication 2, dans laquelle ledit composé est la mésochlorine $e_6$, la bactériochlorine $e_6$, la desvinyl-2 chlorine $e_6$, la mésochlorine $e_6$, l'acétyl-2 chlorine $e_6$, la formyl-2 chlorine $e_6$, ou la rhodine $g_7$.

**6.** Composition de la revendication 2, dans laquelle ledit composé peut être activé par une lumière ayant une longueur d'onde d'environ 620 à 760 nm.